# EUROPEAN PATENT SPECIFICATION

(11) **EP 1 289 455 B1**
(45) Date of publication and mention of the grant of the patent: **20.02.2008**
(21) Application number: 01941806.0
(22) Date of filing: 01.06.2001
(51) Int. Cl.: A61F 5/00, A61F 5/37

(54) **PAD FOR VIBRATION DAMPENING AND CARPEL TUNNEL SYNDROME PREVENTION**
KISSEN ZUR VIBRATIONSDÄMPFUNG UND KARPALTUNNEL-SYNDROM-VORBEUGUNG
MANIQUE PERMETTANT L'AMORTISSEMENT DE VIBRATIONS ET LA PREVENTION DU SYNDROME DU CANAL CARPIEN

(30) Priority: 02.06.2000 US 209022 P
(43) Date of publication of application: 12.03.2003
(73) Proprietor: Shock-Tek LLC, Grosse Pointe, MI 48236 (US)
(72) Inventor: SPITZER, A., Robert, Southfield, MI 48076 (US)
(74) Representative: Tombling, Adrian George
(86) International application number: PCT/US2001/017825
(87) International publication number: WO 2001/093788

(56) References cited:
- US-A- 5 810 753
- US-A- 6 006 751

## Description

### BACKGROUND OF THE INVENTION

### FIELD OF THE INVENTION

This invention generally relates to the field of grips and pads for the protection of a median nerve from pressure thereon, dampening of vibration and absorption of shock.

### DESCRIPTION OF RELATED ART

Carpal tunnel syndrome is a common hand condition that occurs by the compression of a median nerve. The transverse carpal ligament forms over the median nerve and compresses it as a result of pressure on the hand, producing symptoms of pain, numbness, weakness, paraesthesia, or atrophy in the territory of the median nerve distal to the course through the carpal tunnel in the wrist. Traumatic injury is exacerbated because of the narrowness of the carpal canal. There is no opportunity for the nerve to be displaced away from any compressive forces. Additionally, compressive injury is exacerbated by the development of edema in the tissues within the canal, which, because of the relatively fixed size, causes added injury due to compression of the nerve within the canal. An additional component of the injury is associated with friction between the nerve and the adjacent tendons caused by repetitive motions that are worsened by compression and further reduce the available space within the canal.

Carpal tunnel syndrome is a particular problem for workers in industries that require manual operations with hand held implements or tools or in office situations wherein a worker is required to spend several hours a day resting their hands on a typewriter or computer terminal. Carpal tunnel syndrome also occurs due to sports related activities.

Two types of injury can occur due to compression of the median nerve. The milder, earlier form is a demyelination of the median nerve within the canal. This type of demyelination occurs early in the course of compressive injury. This form of injury however, is more readily reversible and recovery occurs in four to six weeks after compression is relieved. This form of injury can cause motor weakness due to acute conduction block in the nerve; however, this weakness is readily reversible.

The second major form of injury includes damage to the axons themselves. This form of injury occurs in more severe or prolonged cases and has more significant implications in that it often leads to motor weakness. This type of motor weakness tends to be poorly reversible and often irreversible. Loss of strength in the thenar muscles can lead to major disabilities due to the loss of ability to grip or perform fine dexterous manipulations. This second form of injury is generally seen in long-standing cases, which first clinically manifest in a manner suggestive of the demyelinating form.

If the median nerve is injured at the wrist, as by wounds or by a dislocation of the lunate bone, sensation is usually lost in the skin on the front of the index finger and adjacent part of the thumb and over the back of the distal phalanges of the thumb; index finger, and middle fingers, and is diminished over a large area. The brunt of the paralysis falls on the muscles of the thenar eminence which, in time, flattens and wastes.

Treatment of carpal tunnel syndrome varies according to the severity of the condition. Severe conditions usually require hand surgery to sever the transverse carpal ligament, whereas in less severe cases, a splint may be utilized to immobilize the wrist

in order to prevent or inhibit a person from development carpal tunnel syndrome, a number of gloves and wrist braces have been designed. One such glove is disclosed in United States Patent_Number '4,'850,341 to Fabry et al., issued July 25, 1989, which discloses a glove for inhibiting or preventing carpal tunnel syndrome, which includes a pad configured to cover and protect the median nerve of the wearer's hand. The problem with such a device is that the pad is placed directly over the median nerve and, therefore, transmits pressure- from the external source to the carpal tunnel ligament. With this type of relationship, repetitive finger movements can increase the potential for frictional inJury. Additionally, a pad oriented directly over the median nerve allows for the:direct transmission of pressure to the median nerve.

In .order to overcome the problems associated with the type of glove disclosed in the Fabry et al. patent, a pad design was disclosed in United States Patent Number 5,031,640 to Applicant, issued July 16, 1991 which eliminates the continuous pressure applied directly over the median nerve by providing a recess over the median nerve in a support pad, thereby preventing or eliminating carpal tunnel syndrome. This type of design has proven to be very effective in inhibiting or preventing carpal tunnel syndrome.

Additionally, U.S. Patent No. 6,006,751 to Applicant discloses a glove for preventing carpal tunnel syndrome. The glove assembly is adapted to inhibit or prevent carpal tunnel syndrome. The glove includes a flexible glove body having a front side and a back side that defines a wrist opening and at least one finger opening. The glove further includes a resilient protector secured to the front side of the glove body for preventing the application of pressure to the median nerve. The resilient protector defines a recess extending substantially parallel with both sides of the median nerve.

In addition to the above-described patents, there are many different types of handgrips available that provide a pad for comfort. These pads mainly prevent external vibration and shock from causing injury to the hands and are normally made of a continuous rubber material including grooves that are decorative in nature. Moreover, by their nature, these pads are soft and compressible in order to absorb vibration. More softness means more vibration dampening, but easy compression of the pads.

The currently existing pads themselves do not provide any protection to the median nerve. In fact, there are no pads that eliminate continuous pressure directly over the median nerve and thereby prevent carpal tunnel syndrome, while also dampening vibration and absorbing shock. The use of a soft pad material for vibration damping and absorption is considered in conflict with the need for a non-compressible pad to prevent pressure from being applied to the median nerve. In combination with the previously described patents, a soft vibration damping material fails to protect the median nerve when the material is compressed by an external pressure source onto the palm of the hand. The compressed pad material expands laterally, such that it closes a significant portion of the recess defined in the previously disclosed inventions. Therefore, pressure is transmitted into the recess and the median nerve is not protected. If the pad is compressed sufficiently and in combination with some deformation of the palm itself, the surface of the external pressure is applied to the palm and the protection is lost. This occurs under static load, or transiently during impact or vibratory oscillation.

Accordingly, there is a need for a device that protects the median nerve from external pressure and external vibration and shock.

### SUMMARY OF THE INVENTION

According to the present invention, there is provided a hand pad apparatus for protecting a median nerve, dampening vibration, and absorbing shock including a protecting mechanism for preventing the application of pressure to the median nerve. The protecting mechanism includes parallel cushion portions defining a recess therebetween and a recess maintaining mechanism for maintaining the recess between the cushion portions. The recess maintaining mechanism is defined as a rigid material disposed about the protecting mechanism, proximate to the recess, and it prevents the cushion portions from reducing the size of the recess. Additionally, the present invention provides for a protecting mechanism including a recess that does not reduce in size after the cushion portions of the protecting mechanism are compressed. Further, the present invention provides for a method of combining median nerve protection, vibration dampening, and shock absorbing to inhibit or prevent carpal tunnel syndrome including the steps of providing the hand pad apparatus and disposing the hand pad apparatus onto a hand, while maintaining the recess over the median nerve.

### DESCRIPTION OF THE DRAWINGS

Other advantages of the present invention will be readily appreciated as the same becomes better understood by reference to the following detailed description when considered in connection with the accompanying drawings wherein:
**Figure 1** is a top view of an embodiment of the present invention made of a protecting mechanism including parallel cushion portions defining a recess extending therebetween and a recess maintaining mechanism for maintaining the recess between the cushion portions, thereby preventing the cushion portions from reducing the size of the recess; .
**Figure 2** is a cross-sectional perspective view of an embodiment of the present invention made of the protecting mechanism, cushion portions, recess and recess maintaining mechanism;
**Figure 3** is a top view of an embodiment of the present invention being oriented onto the palm of a hand and having a varied recess;
**Figure 4** is an embodiment of the present invention wherein the hand pad apparatus is disposed within a glove; and
**Figure 5** is an embodiment of the present invention wherein the hand pad apparatus is composed of numerous individual cushion portions and disposed within a glove.

### DETAILED DESCRIPTION OF THE INVENTION

The present invention provides for a hand pad apparatus, generally shown at **10** in the Figures, for inhibiting or preventing carpal tunnel syndrome and for dampening vibration and absorbing shock. Generally, the hand pad apparatus **10** is composed of a protecting mechanism **12** having a configured recess **14** placed within the protecting mechanism **12** itself. The recess 14 is located and placed over and co-axial with a median nerve **16** of a hand **18**. The recess **14** is substantially parallel with the median nerve **16**. In an embodiment of the present invention, the hand pad apparatus **10** is designed solely for the placement directly onto the palm of the hand **18**.

The present invention is applicable for use in any setting including, but not limited to, offices, factories, homes, industries, businesses, sporting events, recreational activities, and any other similar settings requiring the use of hands being in direct contact with tools, instruments, and devices. The hand pad apparatus **10** is well suited in uses dealing with the transmission of mechanical energy such as shock or vibration. Such mechanical energy originates from sources including, but not limited to, mechanical tools, metal bars, and sporting apparatus. Thus, individuals who have pressure applied directly to their hands greatly benefit from the hand pad apparatus **10** disclosed herein.

The protecting mechanism **12** of the hand pad apparatus **10** is a cushioned pad **24** comprising vibration damping and shock absorbing material or compounds including parallel cushion portions **24**. The parallel cushion portions **24** are composed of materials including, but not limited to, elastomeric material, vulcanized elastomeric material, cis polybutadiene rubber, styrene butadiene rubber, rubber, neoprene, ehtylene propylene terpolymer, styrene butadiene, polyethelene, urethane, air, foam, silicone gel, and any other similar vibration dampening and shock absorbing materials known to those of skill in the art. Generally, these materials or compounds have good abrasion, corrosion, and impact resistance properties. Furthermore, open or closed celled materials are adaptable for use in the present invention. Additionally, if desired, the pad materials are cut to have a waffle pattern. Finally, the cushioned pads **24** are lightweight, malleable, and easily cut to be fabricated into various shapes, contours, and sizes in order to be useful in a wide array of applications.

The critical characteristic of the hand pad apparatus **10** is that it protects the median nerve **16** while dampening vibration and absorbing shock. The recess **14** included in the protecting mechanism **12** prevents the application of pressure to the median nerve **16**. The pressure instead is distributed to the cushion portions **24** of the protecting mechanism **12** and other areas of the protecting mechanism **12**. However, as pressure is applied to the hand pad apparatus **10**, the protecting mechanism **12** is compressed. The compression causes lateral expansion and spread of the cushion portions **24** of the protecting mechanism **12**. The expansion is generally uniform so that expansion of the cushion portion **24** of the protecting mechanism **12** into the recess **14** occurs. Thus, the size of the recess **14** is reduced.

The degree of expansion and spreading of the cushion portions **24** of the protecting mechanism **12** varies depending upon the hardness of the materials or compounds composing the protecting mechanism **12.** In applications where vibration absorption is required, materials or compounds that have a high degree of softness is used. As a result, a high degree of lateral spreading, especially into the recess **14** occurs when pressure is applied to the protecting mechanism **12**. Once the recess **14** is reduced in size due to the lateral expansion and spreading of the cushion portions **24** of the protecting mechanism **12**, the opposing, parallel cushion portions **24** of the protecting mechanism **12** narrow to a point of contact with the median nerve 16. Thus, protection of the median nerve **16** does not occur.

The present invention resolves the problem associated with the lateral expansion and spread of the cushion portions **24** of the protecting mechanism **12** by employing a recess maintaining mechanism **22.**

The cushion portion **24** of the protecting mechanism **12** does not significantly expand into the recess **14** and thus the size of the recess **14** is not reduced and protection of the median nerve **16** occurs.

In the embodiment of the present invention utilizing the recess maintaining mechanism **22**, the recess maintaining mechanism **22** contains a layer of rigid material disposed around the recess **14**. The rigid material includes, but is not limited to, plastic, harden rubber, metal,- wood, cloth, thread, the cushion portion **24** itself, and any other similar material that prevents the reduction of the size of the recess **14** over the median nerve **16**. The recess maintaining means **22** prevents the lateral expansion of the cushion portion **24** into the recess **14** when pressure is applied to the protecting mechanism **12**. Expansion of the cushion **24** is forced outwardly therefrom, but inward expansion into the recess **14** is lessened sufficiently and/or totally prevented.

In another embodiment the recess **14** is varied in size depending upon the type, hardness, and thickness of the cushion pad **24** material. Alternatively, the thickness of The pad material is varied according to the type, hardness and width of the recess **14** between the parallel cushion portions **24** of the protecting mechanism **12**. The softness or hardness of the pad material is key in determining the proper dimensions and configuration of the apparatus **10**. Additionally, the amount of applied static and transient loads being applied to the hand pad apparatus **10** is an influential factor. The proper width of the recess **14** and/or thickness of the cushion portions **24** of the protecting mechanism **12** will vary in order to prevent any pressure from being applied to the median nerve **16**.

The appropriate combination of width of the recess **14** and thickness of the pad material is empirically determined for a specific application. The first step in determining the appropriate combination is to determine the specific application, which involves determining the static load pressure and desired degree of vibration damping. Once these factors are determined, an appropriate material is selected according to its hardness or softness. The hardness or softness of the pad material is determined through the use of a durometer or through any method known to those of skill in the art. Generally, a compromise on hardness is necessary to prevent extreme compression of the pad material and subsequent loss of median nerve **16** protection.

After choosing the appropriate pad material, the pad material must be configured to fit various sized hands. Then, the width of the recess **14** and the thickness of the pad material are adjusted to prevent the recess **14** between the parallel cushion portions **24** of the protecting mechanism 12 from becoming narrower than the width of the median nerve **16** at the maximum static pressure, impact pressure, or vibration. The greatest value of the static pressure, impact pressure or vibration is used to ultimately determine the width of the recess **14** and desired thickness of the pad material. In some situations however, rare extreme transient compression is tolerable. Generally speaking, for a soft material, vibration damping is enhanced, but the pad thickness is greater. For materials with a great deal of lateral expansion and spread under pressure, the recess **14** needs to be wider. For those with minimal lateral expansion and spread, the recess **14** is narrowed.

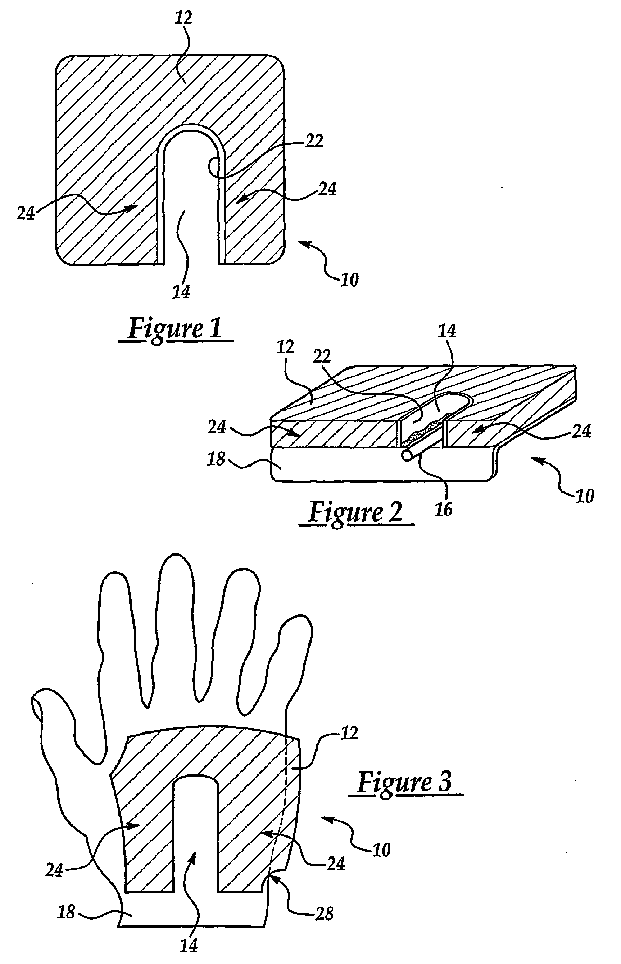

The apparatus is adaptable for use by itself as a hand grip (**Figure 3**) or it is easily adapted to be inserted into a palm surface of a glove assembly **20** to retain its position on the palm of the hand **18** and to maintain the recess **14** over the median nerve **16**. Generally, the glove assembly **20** is a flexible glove body including a front and back side and at least one bridging mechanism **26** disposed between the cushion portions **24** of the protecting mechanism **12** and over the recess **14**. The glove assembly **20** is constructed of a flexible or expandable elastic-type material that conforms to the hand **18** of the user. The flexible material provides a more secure fit for the glove assembly **20**. The flexible material is a fabric that imparts flexibility to the glove assembly **20**, wherein such fabric includes SPANDEX or other similar fabric. However, the glove assembly **20** is made of any material including, but not limited to, cotton, wool, leather, and synthetic materials such as nylons (Kevlar^{™} of DuPont) and polyesters, combinations thereof, and any other similar material known to those of skill in the art.

The bridging mechanism **26** of the glove assembly **20** is constructed of a material that is less resilient than the material that comprises the cushion portions **24** of the protecting mechanism **12**. The bridging mechanism **26** has many forms and shapes necessary to prevent the penetration or infiltration of external objects into the recess **14**, but preferably is "domed" shaped and extends away from the surface of the cushion portion **24**. The glove assembly **20** includes at least one bridge **26**, but usually includes a number of bridges **26** in order to provide adequate coverage of the recess **14** and protection of the median nerve **16.** The bridge **26** is connected to the cushion portions **24** through an elastic portion. The bridge **26** is constructed from any suitable material known to those of skill in the art including, but not limited to, plastic materials such as polystyrene, polyvinyl chloride, polyurethane, as well as metals, utilizing methods known to those of skill in the art of plastic injection molding and forming.

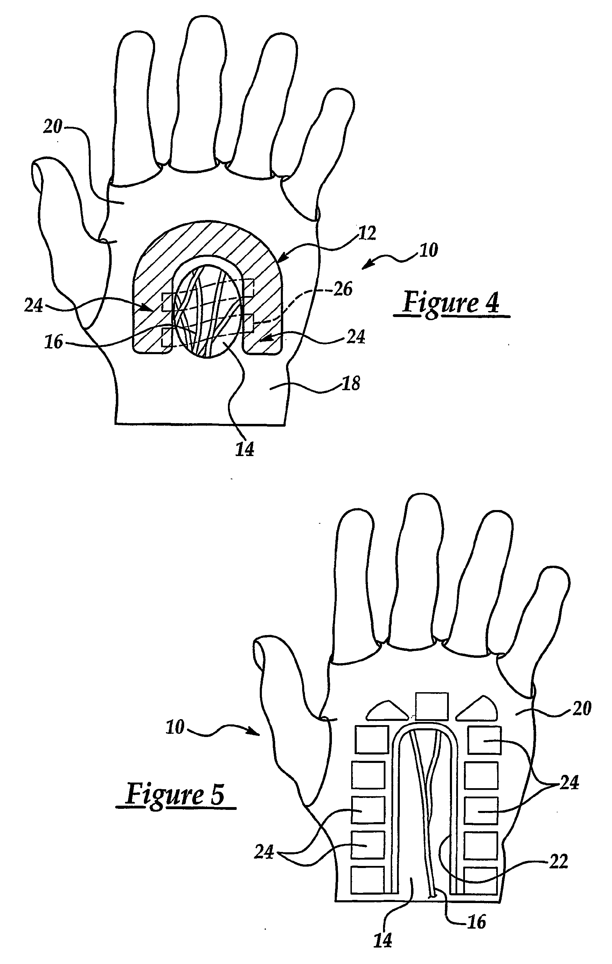

As best illustrated in **Figure 4****,** the protecting mechanism **12** is secured to the palm side of the glove assembly **20.** The protecting mechanism **12** has numerous configurations including, but not limited to, several individual cushions **24** or pads **24** (Figure 5), a single, cushion **24** or pad **24** (**Figures 1 and 2**), a horseshoe shaped pad **24** (**Figure 4****)** having recessed portions in the surface thereof to provide flex areas **28** between the relatively raised portions, and any configuration of the cushion **24** or pad **24** being affixed to the glove assembly **20**.

In operation, the present invention includes the steps of providing the hand pad apparatus **10** including the protecting mechanism **12**, parallel cushion portions **24**, the recess **14**, and the recess maintaining mechanism **22**; and disposing the hand pad apparatus **10** on a hand **18** white maintaining the position of the recess **14** over the median nerve **16**. Additionally, the method further includes disposing the apparatus **10** within a glove assembly **20** to be placed onto the person's hand **18**.

### EXAMPLE

For specific applications exact parameters of pad material hardness, recess 14 width, and pad material thickness is determined empirically. For use in the industrial work glove environment, experiments have shown success in combining the properties of median nerve pr6tection and vibrations using the following typical values:
(1) recess width typically 5 mm to 1 b mm; and
(2) pad material hardness of Shor A 14 - 20.
These values are meant as typical examples for a range of industrial and - sporting applications, but are not meant to represent every possible combination of values that are used. These values were determined primarily with urethane compounds, but other pad materials, such as air filled pads and others described herein, result in different optimal combined values.

Throughout this application, various publications, including United States patents, are referenced by author and year and patents by number.

The invention has been described in an illustrative manner, and it is to be understood that the terminology that has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

## Claims

1. A hand pad apparatus for protecting a median nerve, dampening vibration, and absorbing shock, comprising protecting means (12) for preventing the application of pressure to the median nerve (16) and a recess maintaining means, said protecting means including parallel cushion portions (24) defining a recess (14) therebetween, and wherein said recess maintaining means (22) is defined as a rigid material disposed about said protecting means, proximate to said recess, that prevents lateral expansion of the protecting means into said recess when pressure is applied to the protecting means.

2. The hand pad apparatus according to claim 1, wherein said protecting means is at least one cushion pad made of vibration absorbing material selected from the group consisting essentially of elastomeric material, urethane, foam, polyethylene, rubber, and silicone gel.

3. The hand pad apparatus according to claim 2, wherein said elastomeric material is selected from the group consisting essentially of neoprene, ethylene propylene terpolymer and styrene butadiene.

4. The hand pad apparatus according to claim 2, wherein said rubber is made from materials selected from the group consisting essentially of cis polybutadiene rubber, styrene butadiene rubber, and natural rubber.

5. The hand pad apparatus according to claim 1, wherein said material is made of material selected from the group consisting essentially of plastic, harden rubber, metal, wood, cushion pad, and thread.

6. The hand pad apparatus according to claim 1, wherein said recess is at least as wide as the median nerve and remains at least as wide as the median nerve after pressure is applied to said apparatus.

7. The hand pad apparatus according to claim 1, further defined as a hand grip assembly for placement between a hand and a tool.

8. The hand pad apparatus according to claim 1, wherein said protecting means is disposed within a glove assembly (30).

9. The hand pad apparatus according to claim 8, wherein said glove assembly comprises a flexible glove body including a front side and a back side and at least one bridging means disposed between said cushion portions of said protecting means and over said recess.

10. The hand pad apparatus according to claim 9, wherein said bridging means is made of a plastic material including polystyrene, polyvinyl chloride, and polyurethane.

11. The hand pad apparatus according to claim 9, wherein said bridging means is affixed to said cushion portions of said protecting means.

12. A method of combining median nerve protection, vibration dampening, and shock absorbing to inhibit and prevent carpal tunnel syndrome, comprising the steps of:
preventing the application of pressure to the median nerve (16) by disposing thereabout protection means (12) including parallel cushion portions (24) defining a recess (14) therebetween, the recess being substantially parallel with both sides of the median nerve, and a recess maintaining means (22) defined as a rigid material disposed about said protecting means (12), proximate to said recess, that prevents lateral expansion of the protecting means into said recess when pressure is applied to the protecting means; and
maintaining the recess about the median nerve upon the application of pressure.

13. The method according to claim 12, wherein said disposing step includes disposing the apparatus within a glove (20) to be placed on the person's hand.

## Patentansprüche

1. Handkissenvorrichtung zum Schützen eines Mittelhandnervs zur Vibrationsdämpfung und zur Stoßabsorption, mit einer Schutzeinrichtung (12) zum Verhindern einer Druckwirkung auf den Mittelhandnerv (16) und einer Einrichtung zur Aufrechterhaltung einer Aussparung, wobei die Schutzeinrichtung parallele Polsterabschnitte (24) beinhaltet, die eine dazwischen liegende Aussparung (14) definieren, und wobei die Einrichtung zur Aufrechterhaltung der Aussparung (22) als starres Material definiert ist, welches an der Schutzeinrichtung, unmittelbar an der Aussparung, anliegt und welches die seitliche Ausdehnung der Schutzeinrichtung in die Aussparung hinein verhindert wenn Druck an der Schutzeinrichtung anliegt.

2. Handkissenvorrichtung nach Anspruch 1, wobei die Schutzeinrichtung aus zumindest einem Polsterkissen besteht, welches aus vibrationsabsorbierendem Material gefertigt ist, das aus der nachfolgenden Gruppe ausgewählt ist, bestehend im Wesentlichen aus Elastomermaterial, Urethan, Schaumstoff, Polyethylen, Gummi und Silikongel.

3. Handkissenvorrichtung nach Anspruch 2, wobei das Elastomermaterial aus der nachfolgenden Gruppe ausgewählt ist, bestehend im Wesentlichen aus Neopren, Ethylen-Propylen-Terpolymer und Styrol-Butadien.

4. Handkissenvorrichtung nach Anspruch 2, wobei der Gummi aus Materialien gefertigt ist, die aus der nachfolgenden Gruppe ausgewählt sind, bestehend im Wesentlichen aus Cis-Polybutadien-Gummi, Styrol-Butadien-Gummi und Naturgummi.

5. Handkissenvorrichtung nach Anspruch 1, wobei das Material aus einem Material gefertigt ist, das aus der nachfolgenden Gruppe ausgewählt ist, bestehend im Wesentlichen aus Plastik, Hartgummi, Metall, Holz, Polsterkissen und Zwirnfaden.

6. Handkissenvorrichtung nach Anspruch 1, wobei die Aussparung mindestens so breit ist wie der Mittelhandnerv und mindestens so breit wie der Mittelhandnerv bleibt, nachdem Druck an der Vorrichtung anliegt.

7. Handkissenvorrichtung nach Anspruch 1, des Weiteren definiert als Handgriffanordnung zum Anordnen zwischen einer Hand und einem Werkzeug.

8. Handkissengerät nach Anspruch 1, wobei die Schutzeinrichtung innerhalb einer Handschuhanordnung (20) angeordnet ist.

9. Handkissenvorrichtung nach Anspruch 8, wobei die Handschuhanordnung einen flexiblen Handschuhkörper, der eine Vorderseite und eine Rückseite beinhaltet, und zumindest eine Brückeneinrichtung umfasst, die zwischen den Polsterabschnitten der Schutzeinrichtung und über der Aussparung angeordnet ist.

10. Handkissenvorrichtung nach Anspruch 9, wobei die Brückeneinrichtung aus einem Plastikmaterial gefertigt ist, welches Polystyrol, Polyvinylchlorid und Polyurethan beinhaltet.

11. Handkissenvorrichtung nach Anspruch 9, wobei die Brückeneinrichtung an den Polsterabschnitten der Schutzeinrichtung befestigt ist.

12. Verfahren zum Kombinieren des Schutzes des Mittelhandnervs, der Vibrationsdämpfung und der Stoßabsorption zum Verhindern und Vorbeugen des Karpaltunnelsyndroms, mit folgenden Schritten:
Verhindern von Druckwirkung auf den Mittelhandnerv (16) durch dortige Anbringung einer Schutzeinrichtung (12), die parallele Polsterabschnitte (24), die eine dazwischen liegende Aussparung (14) definieren, wobei die Aussparung im Wesentlichen beidseitig parallel zum Mittelhandnerv vorgesehen ist, und eine Einrichtung zur Aufrechterhaltung der Aussparung (22) aufweist, die als starres Material definiert ist, das um die Schutzeinrichtung (12) unmittelbar an der Aussparung angebracht ist, die die seitliche Ausdehnung der Schutzeinrichtung in die Aussparung hinein verhindert, wenn Druck an der Schutzeinrichtung angelegt wird; und
Aufrechterhalten der Aussparung um den Mittelhandnerv bei Druckeinwirkung.

13. Verfahren nach Anspruch 12, wobei der Schritt des Anordnens die Anordnung der Vorrichtung innerhalb eines Handschuhs (20) zum Anlegen an einer Hand einer Person umfasst.

## Revendications

1. Manique pour protéger un nerf médian, amortir des vibrations et amortir des chocs, comprenant un moyen (12) de protection pour empêcher l'application d'une pression sur le nerf médian (16) et un moyen de maintien d'un évidement, ledit moyen de protection comprenant des portions (24) de coussin d'amortissement parallèles définissant un évidement (14) entre elles, et dans laquelle ledit moyen (22) de maintien d'un évidement est défini comme étant un matériau rigide disposé autour dudit moyen de protection, à proximité dudit évidement, et empêche une expansion latérale du moyen de protection dans ledit évidement lorsqu'une pression est appliquée sur le moyen de protection.

2. Manique selon la revendication 1, dans laquelle ledit moyen de protection est au moins un coussin amortisseur constitué d'un matériau amortisseur de vibrations sélectionné dans le groupe composé essentiellement d'élastomère, d'uréthane, de mousse, de polyéthylène, de caoutchouc et de gel silicone.

3. Manique selon la revendication 2, dans laquelle ledit élastomère est sélectionné dans le groupe composé essentiellement de néoprène, de terpolymère éthylène-propylène et de styrène-butadiène.

4. Manique selon la revendication 2, dans laquelle ledit caoutchouc est constitué de matériaux sélectionnés dans le groupe composé essentiellement de caoutchouc cis-polybutadiène, de caoutchouc butadiène-styrène et de caoutchouc naturel.

5. Manique selon la revendication 1, dans laquelle ledit matériau est constitué d'un matériau sélectionné dans le groupe composé essentiellement de plastique, de caoutchouc durci, de métal, de bois, de coussin amortisseur et de fil.

6. Manique selon la revendication 1, dans laquelle ledit évidement est au moins aussi large que le nerf médian et reste au moins aussi large que le nerf médian après qu'une pression a été appliquée sur ledit dispositif.

7. Manique selon la revendication 1, définie en outre comme un ensemble de prise pour un placement entre une main et un outil.

8. Manique selon la revendication 1, dans laquelle ledit moyen de protection est disposé dans un ensemble gant (30).

9. Manique selon la revendication 8, dans laquelle ledit ensemble de gant comprend un corps de gant souple comprenant une face avant et une face arrière et au moins un moyen d'attache disposé entre lesdites portions de coussin d'amortissement dudit moyen de protection et sur ledit évidement.

10. Manique selon la revendication 9, dans laquelle ledit moyen d'attache est constitué d'un matériau plastique comprenant du polystyrène, du polychlorure de vinyle et du polyuréthane.

11. Manique selon la revendication 9, dans laquelle ledit moyen d'attache est relié auxdites portions de coussin dudit moyen de protection.

12. Procédé de combinaison d'une protection d'un nerf médian, d'un amortissement de vibrations et d'un amortissement de chocs afin de prévenir et d'empêcher un syndrome du canal carpien, comprenant les étapes consistant à :
empêcher l'application d'une pression sur le nerf médian (16) en positionnant autour de celui-ci un moyen (12) de protection comprenant des portions (24) de coussin parallèles définissant un évidement (14) entre elles, l'évidement étant quasiment parallèle aux deux côtés du nerf médian, et un moyen (22) de maintien d'un évidement défini comme étant un matériau rigide disposé autour dudit moyen (12) de protection, à proximité dudit évidement, qui empêche une expansion latérale du moyen de protection dans ledit évidement lorsqu'une pression est appliquée sur le moyen de protection ; et
maintenir l'évidement au-dessus du nerf médian suite à l'application d'une pression.

13. Procédé selon la revendication 12, dans lequel ladite étape de positionnement comprend le positionnement du dispositif dans un gant (20) à placer sur la main d'une personne.
